Europäisches Patentamt

European Patent Office

Office européen des brevets

(19)

(11) Publication number: **0 110 580**
**A2**

(12) # EUROPEAN PATENT APPLICATION

(21) Application number: 83306651.7

(22) Date of filing: 01.11.83

(51) Int. Cl.³: **B 01 D 31/00**, **C 02 F 1/00**,
**C 02 F 1/44**, **A 61 L 2/02**

(30) Priority: 03.11.82 US 438825

(43) Date of publication of application: 13.06.84
Bulletin 84/24

(84) Designated Contracting States: DE FR GB IT SE

(71) Applicant: **Akzona Incorporated, Asheville North Carolina 28802 (US)**

(72) Inventor: **Robinson, James Robert, 3326 Dyer Road, Livermore California 94550 (US)**

(74) Representative: **Allard, Susan Joyce et al, BOULT, WADE & TENNANT 27 Furnival street, London EC4A 1PQ (GB)**

(54) Improved process for removing pyrogens utilizing a hydrophobic microporous membrane.

(57) There is disclosed an improved process for removing pyrogens from a feed liquid which is water or an aqueous solution containing the pyrogens, at a concentration in the range of from 0.1 to 50 ng/ml, comprising the steps of contacting the feed side of a specified hydrophobic microporous membrane with the feed liquid, allowing the liquid to pass through the membrane, and recovering the liquid from the filtrate side of the membrane, these steps being terminated before the membrane becomes saturated with pyrogens.

There is also disclosed an improved process for simultaneously removing pyrogens and particulate matter from a feed liquid which is water or an aqueous solution containing the particular matter and containing the pyrogens, at a concentration in the range of from 0.1 to 50 ng/ml, comprising the steps of contacting the feed side of a specified hydrophobic microporous membrane with the feed liquid, allowing the liquid to pass through the membrane, and recovering the liquid from the filtrate side of the membrane, these steps being terminated before the membrane becomes saturated with pyrogens.

The membrane used in the processes of the present invention is characterized by being made of polypropylene, being substantially skinless, and having (a)   a bubble point in the range of from 25 to 50 psi;
(b)   a thickness in the range of from 3 to 7 mils;
(c)   a nitrogen flow rate of at least 0.5 litres per square centimetre-minute;
(d)   a burst strength of at least 10 psi; and
(e)   an S value of about 15, or less.

EP 0 110 580 A2

0110580

IMPROVED PROCESS FOR REMOVING PYROGENS UTILIZING

A HYDROPHOBIC MICROPOROUS MEMBRANE

This invention relates to a method for removing pyrogens, as well as particulate matter larger than about 0.1 micron, from water or aqueous solutions containing the same.

Pyrogens are generally considered to be a class of materials which have bacterial by-products composed of protein organic matter or complex polysaccharides, which are of a fever producing nature. These pyrogens are produced by certain bacteria which are present in water during distillation and subsequent storage. During sterilization the bacteria are killed, leaving their residue and decomposition products in the water. Pyrogens are believed to be primarily responsible for the majority of reactions which occur subsequent to intravenous injections. With respect to mammals, the entity primarily responsible for pyrogenic reactions is the lipopolysaccharide from Gram-negative bacteria.

Various methods have existed for removing pyrogens

from, for example, water for injection, such as distillation and reverse osmosis treatment. Likewise, a pinhole-free asymmetric ultrafiltration membrane has also been used to remove pyrogens from water or aqueous solutions. In U.S. Patent No. 4,261,834, there is disclosed a composite of two ultrafiltration membranes which may be used to remove pyrogens from water for injection. The composite may be produced by placing the asymmetric membranes in position such that their respective skins are in intimate contact with each other so that pinhole-free portions of one membrane can block pinholes in the other membrane. It is indicated in said patent that using such skin-to-skin composites, five orders of magnitude of pyrogen removal may be accomplished.

Among other disadvantages, filtration through reverse osmosis or ultrafiltration membranes is a timely process and still suffers from the occasional pinhole defect contamination problem. Recently, it has been discovered that a hydrophobic microporous membrane can be used to remove pyrogens from a feed liquid. Said process is satisfactory, but it is always desirable to have the capability to remove pyrogens even more efficiently.

We have now developed an even more effective means for removing pyrogens from water or an aqueous solution at a reasonable flow rate.

There is now provided an improved process for removing pyrogens from a feed liquid which is water or an aqueous solution containing the pyrogens, at a concentration in the range of from 0.1 to 50 ng/ml, which process comprises contacting the feed side of a hydrophobic microporous membrane with the feed liquid, allowing the liquid to pass through the membrane, and recovering the liquid from the filtrate side of the membrane, these steps being terminated before the membrane becomes saturated with pyrogens, the hydrophobic microporous membrane being made of polypropylene, being substantially skinless, and having (a) a bubble point in the range of from 25 to 50 psi; (b) a thickness in the range of from 3 to 7 mils; (c) a nitrogen flow rate of at least 0.5 litres per square centimetre-minute; (d) a burst strength of at least 10 psi; and (e) an S value of about 15, or less.

There is also provided an improved process for simultaneously removing pyrogens and particular matter from a feed liquid which is water or an aqueous solution containing said particulate matter and containing said pyrogens, at a concentration in the range of from about 0.1 to 50 ng/ml, which process comprises contacting the feed side of a hydrophobic microporous membrane with the feed liquid, allowing the liquid to pass through the membrane, and recovering the liquid from the filtrate side of the membrane, these steps being terminated before the membrane becomes saturated with pyrogens, the hydrophobic microporous membrane being made of polypropylene, being substantially skinless, and having (a) a bubble point in the range of from 25 to 50 psi; (b) a thickness in the range of from 3 to 7 mils; (c) a nitrogen flow rate of at least 0.5 litres per square centimetre-minute; (d) a burst strength of at least 10 psi; and (e) an S value of about 15, or less.

The present invention is described hereinbelow with reference to the accompanying drawings, a brief description of which is given below.

Figure 1 is a diagram of an apparatus for carrying out a method for manufacturing a membrane for use in the present invention.

Figure 2 is a scanning electron photomicrograph of a membrane used in the present invention at a magnification of 10,000.

Figure 3 is a scanning electron photomicrograph of a 0.2 micron polypropylene membrane of the prior art.

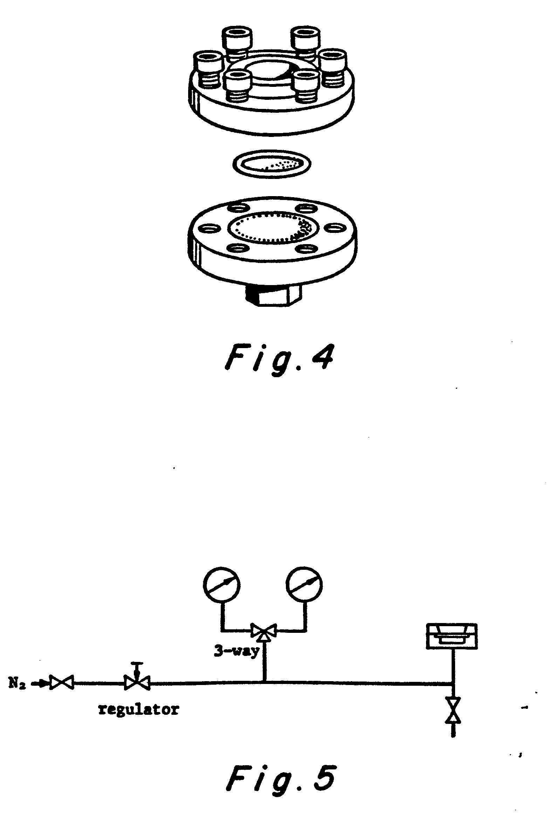

Figure 4 is a diagram of a test filter holder used in a bubble point testing apparatus.

Figure 5 is a diagram of an apparatus used to measure bubble point.

Figure 6 is a diagram of an apparatus used to measure burst strength.

Figure 7 is a diagram of an apparatus used to measure nitrogen flow rate.

As indicated, it was previously found that hydrophobic microporous membranes may accomplish a feat which is quite surprising considering the nature of said membranes. Microporous membranes generally possess a porosity in the size range from about 0.05 to about 5 microns and such porosity is many orders of magnitude larger than pyrogens and thus would not appear to be efficacious in their removal. However, it previously has been discovered that such hydrophobic microporous membranes are quite efficient in removing pyrogens, until the membrane becomes saturated with pyrogens. The removal of pyrogens after such a saturation level has been surpassed is not effective and will result in contamination of the filtrate with pyrogens. Now, it has been discovered that by using a very specific hydrophobic microporous membrane, it is possible to obtain even better pyrogen removal.

It is advantageous that by utilizing the process of the present invention not only can pyrogens be removed from water or an aqueous solution, but simultaneously such water or solution may undergo microporous filtration, removing particles which are larger than about 0.1 micron in diameter. The present invention therefore affords a

0110580

method for accomplishing two distinct results which in the prior art would require two separate processing steps, usually entailing the use of two separate membranes.

Because the microporous membranes of the present invention contain larger pores than, for example, reverse osmosis or ultrafiltration membranes, flow rates larger than that attainable with such prior art membranes may be employed while efficaciously removing pyrogens from the water or solution being processed. A critical aspect of the present invention is to cease the processing of such water or solution at a time prior to saturating the membrane with pyrogens. The use of microporous hydrophobic membranes also overcomes the potential for contaminating the filtrate due to pinhole defects, as exist in ultrafiltration membranes.

The use of the particular membrane of the present invention is critical to obtain the unexpectedly superior performance over even the 0.2 micron polypropylene membranes used in the prior art. The membranes used in the present invention, as previously indicated, are made of polypropylene and are characterized by having

    (a)   a bubble point in the range of from 25 to 50 psi;

    (b)   a thickness in the range of from 3 to 7 mils;

    (c)   a nitrogen flow rate of at least 0.5 litres per square centimetre-minute;

(d)  a burst strength of at least 10 psi; and

(e)  an S value of about 15, or less.

The membrane used in the present invention may be made by heating a mixture of about 30 percent polypropylene and about 70 percent, N,N-bis(2-hydroxyethyl)tallowamine, by weight, to a temperature and for a time sufficient to form  a homogenous solution, casting or extruding said solution at a thickness in the range of from 7 mils, onto a chill roll maintained at a temperature in the range of from 50 to 80°C, allowing the solution to solidify on the chill roll to form a solid sheet, removing the solid sheet from the chill roll and removing at least a substantial portion of the liquid from the sheet to form the microporous polypropylene membrane.

## Bubble Point/Maximum Pore Value

As indicated, the present invention provides a microporous polypropylene membrane which has a unique combination of properties, as contrasted with typical prior art polypropylene membranes.

The bubble point is of primary significance in characterizing the membrane of the present invention, When used in this application the term "bubble point" means the bubble point determined with isopropyl alcohol. From the analytically determined bubble point, one may

calculate a maximum pore-size value for the membrane. The maximum pore-size value is directly related to the rated pore-size of the membrane. For a 0.1 micron rated membrane, the maximum pore-size value should be from about 0.2 micron to about 0.4 micron, with about 0.3 micron being most typical. This translates to a bubble point of from about 50 psi to about 25 psi, with about 30 psi being typical. By contrast for a 0.2 micron rated membrane, the maximum pore-size value should be about 0.4 micron to about 0.65 micron, preferably about 0.5 micron to about 0.6 micron.

To determine the bubble point for a membrane the following technique is employed (adapted from ASTM method F 316).

The necessary apparatus and reagents are:

1. Isopropyl alcohol, ACS reagent grade.

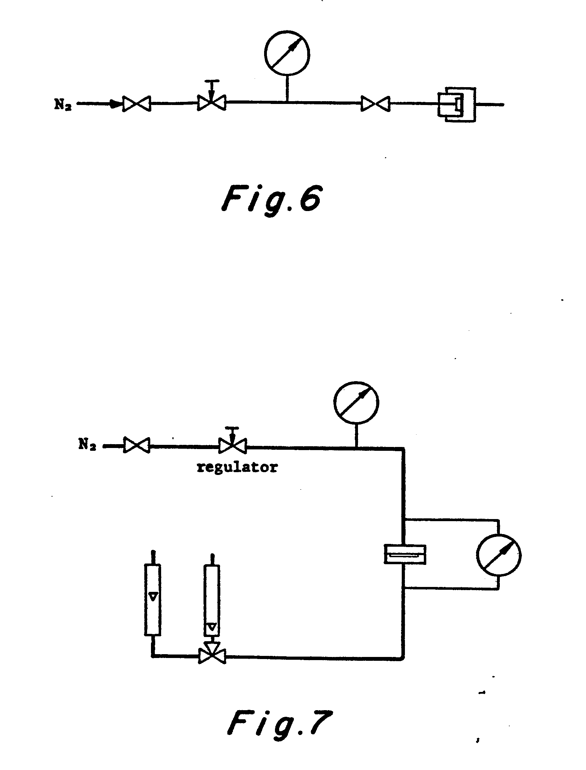

2. Test filter holder consisting of a 47-mm highpressure stainless steel filter holder (Millipore Cat. No. XX45-047-00 or equivalent), with a 3.50-cm opening machined in the top half and 47-mm downstream support screen (Millipore XX42-047-09 or equivalent); see Figure 7.

3. Stainless steel forceps with smooth rounded tips, Millipore XX62-000-06 or equivalent.

4. Mirror-backed test gauges, 0-30 psi and 0-100 psi.

5. Pressure regulator, Bellofram Type 10-B, 2-120 psi (or other appropriate range) or equivalent.

6. 3-way ball valve; other valves, fittings, and tubing as required.

7. 47-mm arch punch and hammer.

8. Petri dish or other liquid container.

9. Source of clean compressed nitrogen.

10. Lighted magnifier.

The procedure is as follows:

1. Assemble the apparatus as shown in Figure 8.

2. Set the nitrogen supply pressure to the apparatus at ~ 10 psi above the expected bubble point or 100 psi.

3. Using the punch, cut five 47-mm discs across the width of the sample.

4. Soak each disc in isopropyl alcohol in the petri dish for 15 seconds or until completely wet.

5. With the forceps, place the wet filter disc on the lower half of the filter holder with the dull side of the disc facing down. Put a few drops of isopropyl alcohol on the disc, then add the support screen (side with large holes in contact with the disc) and the top half of the filter holder. Tighten the bolts securely.

0110580

6.  Pour isopropyl alcohol into the reservoir formed by the hole in the top half of the filter holder to a depth of ∼ 1 cm.

7.  Select the 0-30 psi gauge with the 3-way valve.

8.  Observe the disc through the magnifier while slowly increasing the pressure until the first steady stream of small bubbles is observed rising through the isopropyl alcohol. Record the pressure (to 0.1 psi) at which bubbles appear. The stream of bubbles which marks the bubble point should come from the center portion of the disc instead of the edges where the disc is clamped.

9.  If bubbles have not been observed when the pressure is at 27-28 psi, change the 3-way valve to the 0-100 psi gauge to continue increasing the pressure.

10. After the bubble point has been recorded, reduce the pressure with the regulator and turn off the nitrogen supply to the apparatus.

11. Vent the filter holder by opening the valve at the bottom of the holder.

12. Remove the isopropyl alcohol from the reservoir with a pipette.

13. Disassemble the holder, remove the filter disc and inspect it for obvious holes or defects. Note any defects found.

14. Continue testing as required.

15. Calculate the maximum pore diameter according to the equation $d(\mu m) = \dfrac{0.415\,Y}{BP\ (psi)}$ , where Y = surface tension in dynes/cm. For isopropyl alcohol, the equation reduces to $d(\mu m) = \dfrac{9.02}{BP\ (psi)}$ , for temperatures of 20-25$^{O}$C.

### Thickness

As stated previously, the thickness of the membranes of the present invention is from about 3 to about 7 mils, preferably from about 3.5 to about 4 mils. To determine the thickness, the necessary apparatus is a Starret No. 1015A-431 portable dial hand gauge. This gauge with 1/4" diameter presser foot exerts a pressure of 2.5 psi (+/- 10%) on the sample during a measurement.

The procedure is as follows:

1. Using the adjustment screw on the dial gauge, adjust the gauge to read zero when no sample is being measured.

2. With the lever, raise and lower the presser foot several times to check that the reading returns to zero each time.

3. Raise the presser foot and slip the sample between the foot and base plate.

4. Lower the presser foot onto the sample gently and read the thickness, estimating to 0.1 mil.

5. Test the sample thickness at five locations across the full width of the membrane sample.

. The thickness of the membranes of the present invention is similar to the thickness of prior art 0.2 micron rated membranes which typically have a thickness of about 6 to about 7 mils.

## Burst Strength

As a practical limitation, a membrane must have the ability to withstand normally encountered processing conditions which usually require that the membrane have a burst strength of at least 10 psi, preferably at least 15 psi. The necessary apparatus to determine burst strength is:

1. 13-mm Swinney membrane filter holder, Millipore SX00-013-00 (no support screen), or equivalent, with outlet enlarged to $\geq$ 7 mm diameter.

2. Regulator, range 2-60 psi; Bellofram type 10-B, or equivalent.

3. Test gauge, 0-30 psi with maximum pointer, Ashcroft Type 1009S, or equivalent.

4. Stainless steel forceps with smooth rounded tips, Millipore XX62-000-06, or equivalent.

5. Source of clean compressed nitrogen.

6. 13-mm punch and hammer.

The procedure is as follows:

1. Set up the apparatus as in Figure 9.

2. Cut five 13-mm discs across the width of the film, being careful to avoid including obviously defective areas of the film in the samples.

3.   Using forceps, insert the disc between the two gaskets of the Swinney cell and screw the cell together tightly.  If the film surfaces are different in appearance, place the film in the cell with the dull side toward the gas inlet.

4.   Set the maximum pointer on the gauge to zero.

5.   Turn on nitrogen and slowly increase the pressure until the membrane bursts.

6.   Turn off the nitrogen and reduce the pressure.

7.   Read and record the maximum pressure, to 0.1 psi.  Reset maximum indicator to zero.

8.   Remove the ruptured disc from the cell and examine.  If the rupture is not the typical star-type burst, note that fact, and test an additional disc.

The burst strength of a typical 0.2 micron rated polypropylene membrane is also usually above 10 psi.

### Nitrogen Flow Rate

In order for any membrane to be useful, it must not only be effective in removing any material present which is larger than a given size, such as 0.1 micron, it must also be capable of accomplishing such a filtration within a reasonable length of time.  One way to project the anticipated filtration rate which may be obtained by using a given membrane is by determining the nitrogen flow rate for the membrane. A high nitrogen flow rate is usually indicative of a structure that has a low resistance to fluid passage, thus making such a membrane desirable for filtration provided that all of the other physical characteristics are suitable. Membranes of the present invention have a nitrogen flow rate of at least 0.5 and preferably at least 0.7, liters per square centimeter-minute.

The necessary apparatus to determine nitrogen flow rate is:

1. Filtered (0.45 μm or 0.2 μm) nitrogen at 30-50 psi.

2. Pressure regulator, Bellofram type 10B, 2-25 psi, or equivalent.

3. Pressure gauge, 0-30 psi.

4. Millipore stainless steel high pressure 47-mm filter holder, Cat. No. XX45-047-00, or equivalent.

5. Filter forceps with unserrated tips, Millipore XX62-000-06, or equivalent.

6. Magnehelic 0-20 psi differential pressure gauge, Dwyer Instruments Cat. No. 2220, or equivalent.

7. One or more flow meters of appropriate capacity (Matheson type 605 with stainless steel float is standard; different tubes and floats may be required for very high or low flows).

The procedure is as follows:

1. Set up the apparatus as shown in Figure 10.

2. Using the arch punch, cut five 47-mm discs from each membrane sample to be tested.

3. Using forceps, place the disc in the filter holder with the dull side upstream. Put the top half of the filter holder in place and tighten securely.

4. Open valve B to the desired flow meter (is more than one).

5. Open valve A to admit nitrogen to the apparatus.

6. With the regulator, adjust the pressure to 10 psi on the Magnehelic gauge.

7. Read the flow meter at the middle of the ball and record. If the first is in the bottom or top 10% of the tube, switch to a higher or lower range flow meter if available.

8. Close valve A, disassemble the filter holder and remove the membrane filter. Install the next disc and continue testing.

## Sharpness

Another means of characterizing the membranes of the present invention is by a sharpness factor, "S". The S factor is determined by analyzing a mercury intrusion curve for the given membrane. All mercury intrusion data discussed in this application was determined by use of a Micromeritics Mercury Penetration Porosimeter, Model 910 series. The S value is defined as the ratio of the pressure at which 85 percent of the mercury penetrated to the pressure at which 15 percent of the mercury penetrated. This ratio is a direct indication of the variation in pore diameter across the central 70 percent of the pores in any given sample, as pore diameter is equal to 176.8 divided by the pressure in psi.

The S value, then, is a ratio of the diameter of the pores at which 15 percent of the mercury has intruded to the diameter of the pores at which 85 percent of the mercury has intruded. The range for 1 to 15 percent and 85 to 100 of mercury intrusion is ignored in determining the S factor. The range from 0 to 15 percent is ignored as penetration in this range may be due to cracks introduced into the material as a result of the freeze-fracturing to which the material was subjected prior to performing the mercury intrusion study. Also, the range from 85 to 100 percent is ignored as data in such a range may be due to compression of the sample rather than to actual penetration of the mercury into the pores.

Characteristic of the range of pore sizes exhibited by the membranes of the present invention, the usual S value for such structures is usually less than about 15 and thus is in the range of from about 1 to 15, with a range of about 8 to 14 being typical, and a value of about 11 being representative.

## Scanning Electron Photomicrographs

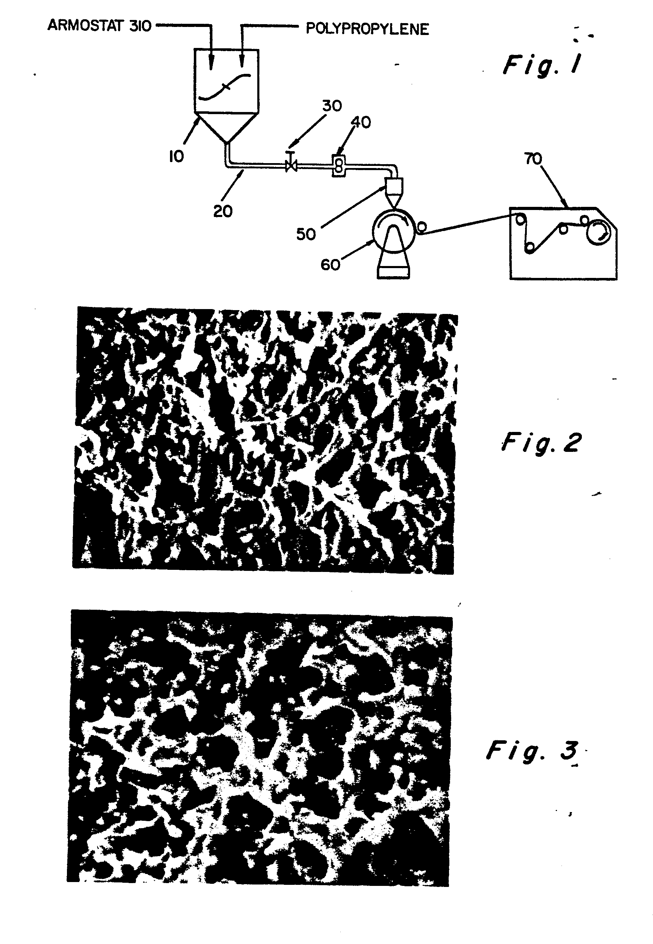

One further way to compare the porosity of a membrane used in the present invention to that of prior art membranes, is through the use of scanning electrion photomicrographs (SEM's). Figure 2 is an SEM of a membrane used in the present invention at a magnification of 10,000 whereas Figure 3 is an SEM of a 0.2 micron membrane of the prior art, also a magnification of 10,000. By comparing the two figures, it is apparent that the membrane used in the present invention possesses a smaller average pore-size.

## PROCESS DETAILS

The process for making the membrane used in the present invention is based upon the process described in U.S. Patent No. 4,247,498, which process is described in the art as a thermally induced phase separation process. To apply the method of U.S. Patent No. 4,247,498 for the production of membranes, such as those rated 0.2 microns, a batch operating system has been employed in the art.

Figure 1 shows a typical configuration for the batchwise production of membranes. In Figure 1, N,N-bis(2-hydroxyethyl)tallowamine, such as that sold by Armak Company, Chicago, Illinois, under the trademark Armostat 310, (ARMOSTAT is a Registered Trade Mark) is introduced into mixing tank 10, which is connected to casting box 50

- 18 -

0110580

through conduit 20. A valve 30 and a pump 40 are usually located along conduit 20. The mixing tank is heated by any convenient means and the temperature of the amine is raised to about $200^{\circ}C$. Polypropylene, as sold by Phillips Petroleum Company, type No. BP-145, MFR 9.5, usually in the form of chips, is introduced into the mixing tank, in an appropriate weight ratio of amine to resin. The mixture is maintained at about $200^{\circ}C$ for about 1.5 hours during which time the polymer dissolves in the amine, forming a homogeneous solution. After said solution has been formed, the solution is pumped into casting box 50 through which the solution flows at an appropriate rate to cast a layer on chill roll 60 having a thickness of about 3 to 7 mils. The space between the chill roll and the lip on the casting box can be adjusted and determines the film thickness. Instead of the casting box, an extruder and die-head may be used to extrude a layer onto the chill roll.

The chill roll temperature is typically at a temperature of about $75^{\circ}C$ when one is making a 0.2 micron rated membrane and rotates at a surface speed of about 15 feet per minute. When the solution contacts the chill roll cooling has started and by the time the solution reaches a temperature of about $170-180^{\circ}C$, phase separation occurs. Upon further cooling, the polypropylene solidifies. After the solidification has occurred, the solid film is removed from the chill roll 60 via a take-up system 70.

When the foregoing system is employed to manufacture membranes within the scope of the present invention, approximately 28 pounds of N,N-bix(2-hydroxyethyl)-tallowamine is mixed with about 9.3 pounds of polypropylene in mixing tank 10. The pump 40, conduit 20 and casting box 50 are maintained at 200°C by any suitable means. The chill roll 60 is maintained at about 60°C and is rotated at a surface speed of about 15 feet per minute.

It is often desirable to treat the solidified film, after winding into a roll, by submerging the roll in a bath of N,N-bis(2-hydroxyethyl)tallowamine, maintained at a temperature of about 135°C for about 3 hours.

Any suitable means may be employed to extract the amine from the solidified film, as by soaking in consecutive baths of isopropyl alcohol, to achieve an amine level of less than 0.2 percent in the final membrane.

GENERAL

From the foregoing description it should be apparent that several aspects of the method for making a suitable membrane are critical. The ratio of amine to the weight of polypropylene is critical, as further reduction in the amount of polypropylene may result in a membrane with poor mechanical properties, such as a low burst strength. If the amount of polypropylene is increased, the result may be unacceptable nitrogen and/or water flow rates.

The cooling rate of the solution of amine and polypropylene is critical inasmuch as a rapid cooling rate generally produces a smaller pore-size, and a slower cooling rate results in a larger pore size. As the cooling rate is simply relates to the length of time it takes the solution to solidify after the phase separation has occurred, the primary tool in controlling the cooling rate is the temperature of the chill roll. However, if the temperature of the chill roll is too slow, the result will be a substantial formation of skin on the membrane side in contact with the chill roll surface.

A skin is simply a region which has an apparent polymer density different from that of the remainder of the membrane. For example, often times a skin having an apparent density much higher than that of the remainder of the membrane may be formed. When a very low chill roll temperature is used, the skin may extend from the surface of the membrane in contact with the chill roll, across 20 percent, or more, of the cross-section of the membrane. It would not be unusual to have the skin extend throughout even 30 to 50 percent of the membrane cross-section, and more, if an inappropriate chill roll temperature is employed.

The membrane used in the present invention is

substantially skinless. By the term "substantially skinless", it is meant that the membrane has no more than about 20 percent of its cross-section occupied by a skin layer. Preferably, the skin layer occupies no more than 10 percent of the cross-section, and most preferably, less than 5 percent.

If the chill roll temperature is too warm, proper solidification may not occur in time to remove a solidified sheet from the layer on a continuous basis. Also, as stated, a warm chill roll will result in a slower cooling rate and possibly too large of a pore diameter.

The thickness of the membrane, of course, affects the flow rate properties of the membrane, the thicker the membrane, the lower the flow rate, but having a thin membrane can result in a membrane having poor mechanical properties, such as a low burst strength.

As stated, a critical aspect of the process of the present invention is to terminate the depyrogenation of the feed liquid prior to saturating the membrane with pyrogens. Based upon the results which were obtained in the following examples, it has been calculated that saturation occurs at a level greater than about 2,000, and probably at least about 20,000, micrograms of pyrogens, per $cm^3$ of membrane volume. A typical saturation level would therefore be at least about 2,000 micrograms per $cm^3$ of membrane volume,

preferably at least about 20,000 micrograms per cm³ of membrane volume.

A typical microporous membrane will have a thickness of from about 3 to about 7 mils (76 to 178 micrometers). Thus, a 3.8 mil thick membrane (96.5 micrometers), such as one having a surface of 12.5 cm², which had a pyrogen removal capacity of at least 20 micrograms per cm², would have a saturation point of about 2,074 µg/cm³, or greater. Thus, using such a membrane, the depyrogenation may typically be continued until from about 0.02 to at least about 20 micrograms of pyrogens, or more, have been removed, per cm² of membrane surface area, preferably until at least about 200 micrograms of pyrogens per cm² of membrane surface area has been removed.

With respect to the liquids to be filtered, it is also important that the pyrogen content not be unduly high so that the removal capability of the membrane is not quickly exceeded. In industries such as the pharmaceutical industry, typical solutions to be depyrogenated will contain from about 0.3 to about 30 ng of pyrogens per ml of liquid.

Any particulate matter present in the liquid to be filtered will usually have a particle size less than about 100 microns as such liquids will normally have been subjected to gross filtration, utilizing pre-filtration devices, before ever being subjected to depyrogenation. Also, as previously indicated, the liquid may be pure water or a solution which already contains some dissolved specie, such as a pharmaceutically active material.

The hydrophobic microporous membranes may be employed in any useful physical form. Typical forms are, for example, flat circular discs or pleated cartridges having a membrane thickness of from about 3 to about 7 mils, typically about 3.5 mils.

Another particularly useful form of the membrane is a hollow tube.

The invention will be further described by way of the following nonlimiting examples.

Example 1

To demonstrate the efficacy of the present invention in removing pyrogens, feed solutions were prepared containing various amounts of endotoxin and the concentration of endotoxin in the feed solution and in the filtrate were measured, using the published spectrophotometric procedure of the Associates of Cape Cod, Incorporated, Wood Hole, Massachusetts.

In the experimental apparatus a McGaw 1000 ml siliconized IV bottle was connected to an IV stopper with a vent tube and subsequently to a McGaw No. V1400

disposable IV set, which was in turn connected to a Sartorius stainless steel connector (female lueur/male thread). The connector was then attached to a 47/50 mm Sartorius polycarbonate filter holder which was placed upon and supported by a Wheaton 1000 ml IV bottle of borosilicate glass, siliconized.

Prior to performing the experiment, the IV bottles and the Sartorius stainless steel connector were depyrogenated by dry heat at 180°C for 3 hours. The IV bottle stopper and vent tube and the Sartorius filter holder were depyrogenated by autoclaving in dilue HCl. The pyrogen free disposable IV set was supplied sterile.

Challenge solutions containing approximately 30 ng/ml of endotoxin were prepared and approximately 1000 ml of the various solutions were filtered at a flow rate of about 10.5 ml/minute with the experimental apparatus. The filtrate was collected and the endotoxin level measured.

To demonstrate the advantage of using a membrane as claimed in the present invention over a typical 0.2 micron polypropylene membrane of the prior art, one membrane falling within the scope of the present invention and one membrane falling within the scope of the prior art were subjected to the previously described endotoxin challenge. The two membranes had the properties shown in Table I.

TABLE I

| MEMBRANE | BUBBLE POINT | THICKNESS | | N$_2$FLOW 1/min-cm$^3$ | BURST (psi) |
| | | micrometers | mils | | |
|---|---|---|---|---|---|
| Invention | 29.2 | 96 | 3.8 | 0.79 | 15.6 |
| Prior Art | 17.7 | 157.1 | 6.2 | 1.29 | -- |

The membrane of the present invention was made in accordance with the standard procedure described above, utilizing a chill roll temperature of 60°C and a cloth wiper blade bar, to alleviate the sticking together of layers of solidified film. The wiper bar was installed on the chill roll at a location just prior to the casting bar.

## TABLE II

| PORE SIZE (micron) | INTENDED CHALLENGE CONC. (NG/ML) | MEASURED CHALLENGE CONC. (NG/ML) | MEASURED PASSAGE CONC. (NG/ML) | TOTAL CHALLENGE (µG) | TOTAL PASSAGE (µG) | TOTAL CHALLENGE PER UNIT AREA (µG/CM²) | TOTAL CHALL. / TOTAL PASSAGE =RV | $LOG_{10}(RV)$ |
|---|---|---|---|---|---|---|---|---|
| 0.1 | 30 | 33.9 | .126 | 33.9 | .126 | 2.71 | 269 | 2.430 |
|  |  | 55.0 | <.010 | 55.0 | <.010 | 4.40 | >5500 | 3.740 |
|  |  |  |  |  |  |  |  | Ave. 3.460 |
| 0.2 | 30 | 35.4 | 5.03 | 31.7 | 5.03 | 2.54 | 6.30 | .799 |
|  |  | 54.0 | 3.99 | 54.0 | 3.99 | 4.32 | 13.5 | 1.130 |
|  |  |  |  |  |  |  |  | Ave. 0.996 |

From the results shown in Table II it appears that a $\text{Log}_{10}$ (RV), "LRV", value of from about 2.43 to 3.74 may be attained with the membrane of the present invention when the challenge concentration is at a level from greater than 30 ng/ml, or less, and the total amount of endotoxin with which the membrane is challenged is at some value from greater than 4.40 $\mu g/cm^2$ of membrane surface, or less or in terms of membrane volume from greater than about 456 $\mu g/cm^3$, or less.

CLAIMS:

1.   An improved process for removing pyrogens from a feed liquid which is water or an aqueous solution containing the pyrogens, at a concentration in the range of from 0.1 to 50 ng/ml, which process comprises contacting the feed side of a hydrophobic microporous membrane with the feed liquid, allowing the liquid to pass through the membrane, and recovering the liquid from the filtrate side of the membrane, these steps being terminated before the membrane becomes saturated with pyrogens, characterized in that the hydrophobic microporous membrane is made of polypropylene, is substantially skinless, and has (a) a bubble point in the range of from 25 to 50 psi; (b) a thickness in the range of from 3 to 7 mils; (c) a nitrogen flow rate of at least 0.5 litres per square centimetre-minute; (d) a burst strength of at least 10 psi; and (e) an S value of about 15, or less.

2.   A process as claimed in claim 1 wherein the membrane has a bubble point of about 30.

3.   A process as claimed in claim 1 or claim 2 wherein the membrane has a thickness of about 3.5 mil.

4.   A process as claimed in any one of the preceding claims wherein the steps are continued until from 0.02 to 200 micrograms of pyrogens per $cm^2$ of membrane surface area have been removed.

5. A process as claimed in claim 4 wherein the steps are continued until at least 20 micrograms of pyrogens per $cm^2$ of membrane surface area have been removed.

6. A process as claimed in any one of claims 1 to 5, wherein the steps are continued until at least about 2,000 micrograms of pyrogens per $cm^3$ of membrane volume have been removed.

7. An improved process for simultaneously removing pyrogens and particulate matter from a feed liquid which is water or an aqueous solution containing the particulate matter and containing the pyrogens, at a concentration in the range of from 0.1 to 50 ng/ml. which process comprises contacting the feed side of a hydrophobic microporous membrane with the feed liquid, allowing the liquid to pass through the membrane, and the liquid is recovered from the filtrate side of the membrane, these steps being terminated before the membrane becomes saturated with pyrogens, characterized in that the hydrophobic microporous membrane is made of polypropylene, is substantially skinless, and has (a) a bubble point in the range of from 25 to 50 psi; (b) a thickness in the range of from 3 to 7 mils; (c) a nitrogen flow rate of at least 0.5 litres per square centimetre-minute; (d) a burst strength of at least 10 psi; and (e) an S value of about 15, or less.

8.    A process as claimed in claim 7 wherein the membrane has a bubble point of about 30.

9.    A process as claimed in claim 7 wherein the membrant has a thickness of about 3.5 mil.

10.    A process as claimed in any one of claims 7 to 9 wherein the steps are continued until from 0.02 to 200 micrograms of pyrogens per cm² of membrane surface area have been removed.

11.    A process of claim 7 wherein the steps are continued until at least about 20 micrograms of pyrogens per cm² of membrane surface area have been removed.

12.    A process as claimed in any one of claims 7 to 9 wherein the steps are continued until at least about 2,000 micrograms of pyrogens per cm³ of membrane volume have been removed.

ARMOSTAT 310 ———————— POLYPROPYLENE

*Fig. 1*

10

30

40

20

70

50

60

*Fig. 2*

*Fig. 3*

*Fig. 4*

3-way

N₂ →

regulator

*Fig. 5*

Fig.6

regulator

Fig.7